# EUROPEAN PATENT APPLICATION

(11) **EP 3 954 249 A1**
(43) Date of publication of application: **16.02.2022**
(21) Application number: 21170114.9
(22) Date of filing: 23.04.2021
(51) Int. Cl.: A45D 34/04, A61F 7/00, A61N 5/06, C02F 1/467

(54) **BEAUTY DEVICE WITH HYDROGEN STERILIZATION WATER SPRAY AND TEMPERATURE STIMULATION FUNCTION**

(30) Priority: 13.08.2020 KR 20200101991
(71) Applicant: Soovon Co., Ltd., Incheon 21315 (KR)
(72) Inventor: OH, Kyung Hee, 10388 Gyeonggi-do (KR)
(74) Representative: Hwang, Jae Suk

(57) **Abstract**

The beauty device according to the present invention comprises by including a mist processing part generating sterilization water by electrolyzing water in a water tank and spraying the generated sterilization water in the form of mists, and a temperature stimulation processing part applying temperature stimulation to skin using an thermoelement. A more convenient and efficient skin care can be made possible because a mist spray function of hydrogen sterilization water and a temperature stimulation function can be simultaneously implemented using a single portable device. The temperature stimulation function can be simply implemented because temperature is controlled using a peltier device.

## Description

### [CROSS-REFERENCE TO RELATED APPLICATION]

This application claims priority to and the benefit of Korean Patent Application No. 10-2020-0101991 filed on August 13, 2020 in the Korean Intellectual Property Office, the entire content of which is incorporated herein by reference.

### [Technical Field]

The teachings in accordance with exemplary and non-limiting embodiments of this invention relate generally to a beauty device with hydrogen sterilization water spray and temperature stimulation function, and more particularly to a portable beauty device configured to perform both a function of spraying hydrogen sterilization water in the form of mists by generating the hydrogen sterilization water through electrolysis and a function of providing temperature stimulation to skins using a thermoelement.

### [Background of the Invention]

Recently, not only women but also men are paying special attention to beauty to cherish a beautiful appearance.

In general, cosmetics including, but not limited to, collagen, nutritional fluid, essence are largely used for beauty that help moisturization, nutrition supply and skin function activation.

Furthermore, mist suppliers are used for skin beauty, where the mist suppliers release or blow out mists in fine particulate state.

Mist suppliers may be used for various purposes including, but not limited to, moisturization, skin whitening (skin-lightening) effect and nourishment, and also widely used for moisturizing applications to resolve the skin drying.

The Korean registered patent No10-2020-0086433 discloses a portable mist device configured to be conducive to beauty by generating hydrogen sterilization water using electrolysis, and spouting the generated hydrogen sterilization water in the form of mists through ultrasonic oscillator.

Meantime, various factors may be used for skin beauty including but not limited to not only mists but also temperature, specific wavelength of light, current and electric fields such as galvanic or EP (Electropolation) and physical massage through vibration.

Particularly, temperature stimulations such as cold stimulation and warm stimulation adequately applied to skin may provide a preferable effect to skin beauty.

However, the conventional beauty devices that provide sterilization water in the form of mists through electrolysis like the Korean registered patent No.:10-2020-0086433 cannot be coupled to temperature stimulation function.

Still furthermore, although thermoelement such as peltier device may be used in consideration of the characteristic as a portable device and ease in temperature adjustment or control when the temperature stimulation function is included in the beauty device, the peltier device may be damaged due to temperature easily rising to high temperature, and therefore, an adequate cooling method must be sought after within a limitation as a portable device.

### [Related Technical Documents]

### [Patent Documents]

Korean registered patent No.:10-2020-0086433 (Published on July 17, 2020)

### [Summary of the Invention]

### [Technical Subject]

The present invention is devised to solve the aforementioned disadvantages/problems and it is an object of the present invention to provide a beauty device configured to embody, in one portable device, both a function of spraying sterilization water in the form of mists by using electrolysis and a function of providing temperature stimulation to skins using a thermoelement, and to adequately perform the cooling of thermoelement as well.

### [Technical Solution]

In one aspect of the present invention, there may be provided a beauty device having a function of hydrogen sterilization water spray and a function of providing temperature stimulation to skins, the device comprising:
a mist processing (treatment) part generating sterilization water by electrolyzing water in a water tank and spraying the generated sterilization water in the form of mists;
a temperature stimulation processing (treatment) part applying temperature stimulation to skin using a thermoelement;
a power supply part supplying an electric power;
an input part receiving an operation command; and
a controller controlling the mist processing part and the temperature stimulation processing part in response to the operation command inputted through the input part, wherein a housing forming an external appearance may be formed in a portable manner.

Preferably but not necessarily, the thermoelement may include a peltier device.

At this time, one surface of the peltier device may be so configured as to thermally contact the water stored in the water tank of the mist processing part.

Preferably but not necessarily, the thermal contact may be realized by a first heat dissipation part of first material directly contacting the water stored in the water tank of the mist processing part and a second heat dissipation part of second material interposed between one surface of the peltier device and the first heat dissipation part.

Preferably but not necessarily, wherein the first material may be formed with a material less polluted than the second material by the sterilization water generated through the electrolysis.

At this time, the first material may include SUS material and the second material may include aluminum material.

Preferably but not necessarily, the second heat dissipation part may be so disposed as to accommodate a cooling liquid therein.

Preferably but not necessarily, the second heat dissipation part may be so disposed as to be tight-sealed by closing a lid after pouring the cooling liquid into an inner space.

Preferably but not necessarily, the housing may include a pillar-shaped handle part graspable by one hand of a user, and a head part protruded from an upper end of the handle part to both lateral surfaces.

At this time, one side of the head part may be disposed with a raw water inlet to allow water to be poured into the water tank of the mist processing part, and the other side of the head part may be disposed with a skin contact member configured to apply a temperature stimulation to skin.

Preferably but not necessarily, the water tank of the mist processing part may be formed in the ' 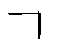 ' shape from the raw water inlet across an upper space of the handle part, and the upper end of the handle part may be formed with a mist outlet toward a direction formed with the raw water inlet.

At this time, the thermal contact of the first heat dissipation part and the second heat dissipation part may be implemented at an area where the water tank is curbed.

Preferably but not necessarily, a surrounding of the skin contact member may be disposed with a transparent window, and a specific wavelength of light useable for skin beauty may be outputted from the transparent window.

### [Advantageous Effects]

According to the present invention, a user can utilize, with a single portable device, a function of spraying hydrogen sterilization water in the form of mists and a function of providing temperature stimulation at the same time, thereby caring skin more conveniently and efficiently.

Particularly, the temperature can be controlled using a peltier device to thereby enable a simple implementation of cool stimulation and warm stimulation functions.

At this time, an excessive temperature rise of peltier device can be adequately restrained to effectively prevent the damage to the thermoelement.

### [Brief Description of Drawings]

FIG. 1 illustrates a beauty device according to an exemplary embodiment of the present invention,
FIGS. 2, 3 and 4 illustrate a housing portably forming a beauty device according to an exemplary embodiment of the present invention,
FIG. 5 illustrates an example where a mist processing part and a temperature stimulation processing part are respectively disposed at both sides of a head part of a housing,
FIG. 6 illustrates a mist processing part according to a detailed exemplary embodiment of the present invention,
FIG. 7 illustrates a temperature stimulation processing part according to a detailed exemplary embodiment of the present invention,
FIG. 8 illustrates a first heat dissipation part directly contacting sterilization water inside a water tank according to an exemplary embodiment of the present invention,
FIG. 9 illustrates a structure of a second heat dissipation part according to a detailed exemplary embodiment of the present invention, and

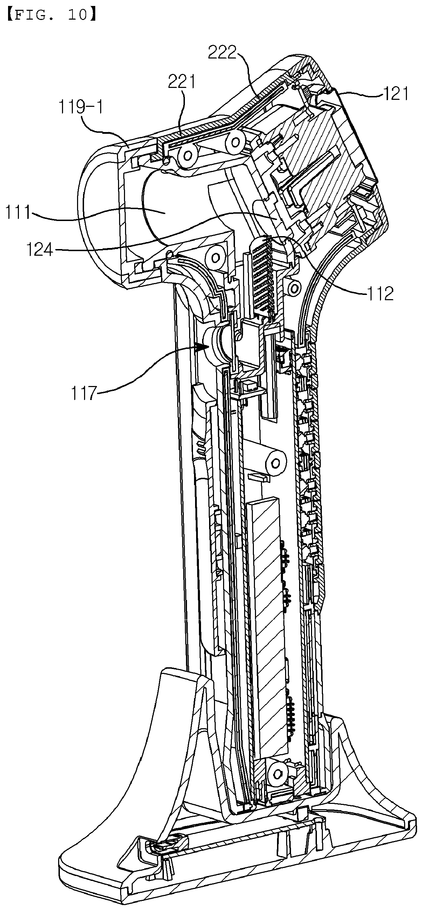

FIG. 10 illustrates a cross-sectional view of a housing according to an exemplary embodiment of the present invention.

### [Detailed Description]

The present invention may be applied with various changes and have several exemplary embodiments, where particular exemplary embodiments will be exemplified in the drawings and described in detail through the detailed description of the present invention.

However, it should be understood that the present invention is not limited to particular embodiments, but encompasses all changes, modifications, equivalents and substitutes included within the ideas and technical scopes of the present invention.

In describing the present invention, detailed descriptions of well-known technologies are omitted for brevity and clarity so as not to obscure the description of the present invention with unnecessary detail.

The terminology used herein is for the purpose of describing exemplary embodiments only and is not intended to be limiting. As used herein, the singular forms may be intended to include the plural forms as well, unless the context clearly indicates otherwise.

It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

It will be understood that, although the terms first, second, etc. may be used herein to describe various elements, these elements should not be limited by these terms. These terms are only used to distinguish one element from another.

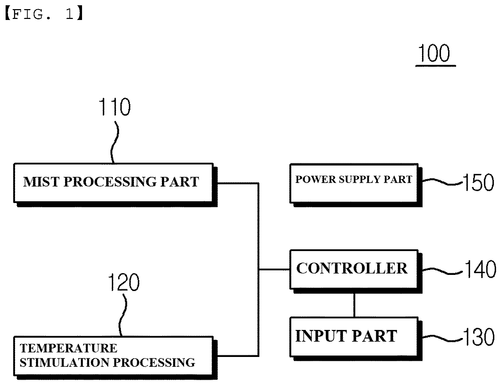

FIG. 1 illustrates a functional block diagram of a beauty device 100 according to an exemplary embodiment of the present invention, and the beauty device 100 may comprise by including a mist processing part 110 generating sterilization water by electrolyzing water in a water tank and spraying the generated sterilization water in the form of mists, a temperature stimulation processing part 120 applying temperature stimulation to skin using an thermoelement, an input part 130 receiving an operation command, and a controller 140 controlling the mist processing part 110 and the temperature stimulation processing part 120 in response to the operation command inputted through the input part 130, and a power supply part 150 supplying an electric power

The input part 130 may receive various operation commands from a user about operations of the beauty device 100. The operation commands and input methods inputted through the input part 130 may be variably configured. However, the present invention is not particularly limited thereto.

For example, the input part 130 may include various switches and buttons related to various functions such as ON/OFF of electric power of beauty device 100, ON/OFF of mist functions, ON/OFF of temperature stimulation functions, cooling stimulation set-up, warm stimulation set-up and temperature set-up.

Although a housing forming an exterior appearance of the beauty device 100 may be variably formed as diverse as possible, it is preferable that the housing be formed in a portable manner to allow a user to hold and use with one hand.

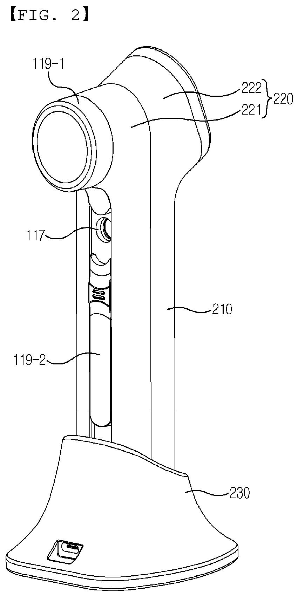

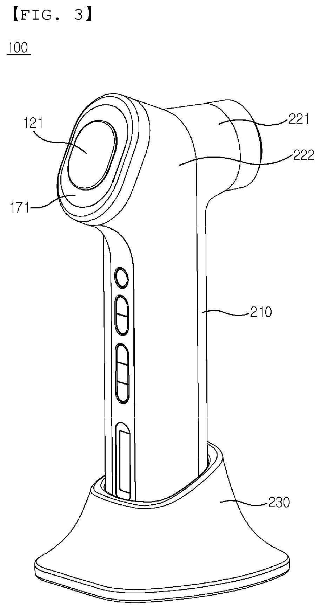

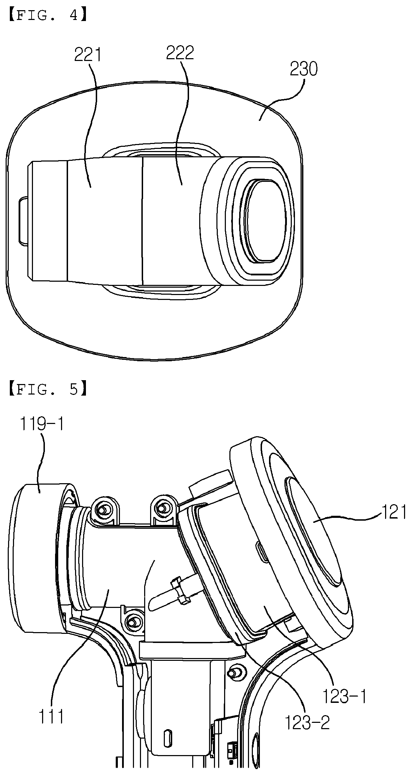

FIGS. 2, 3 and 4 illustrate an example of a housing portably forming a beauty device 100 according to the present invention, where FIG. 2 is a perspective view seen from a direction formed with a mist outlet 117, FIG. 3 is a perspective view seen from a direction from which a skin contact member 121 is seen that contacts a skin of a user to provide a temperature stimulation, and FIG. 4 is a schematic view seen from above a housing.

The said housing may be formed by including a pillar-shaped handle part 210 to allow a user to grasp with one hand, and a head part 220 protruded from an upper end of the handle part 210 toward both lateral surfaces.

Referring to FIG. 5, an area 221 protruded from the head part 220 of the housing to one side may be disposed with various elements forming the mist processing part 110, and an area 222 protruded to the other side may be disposed with various elements forming the temperature stimulation processing part 120.

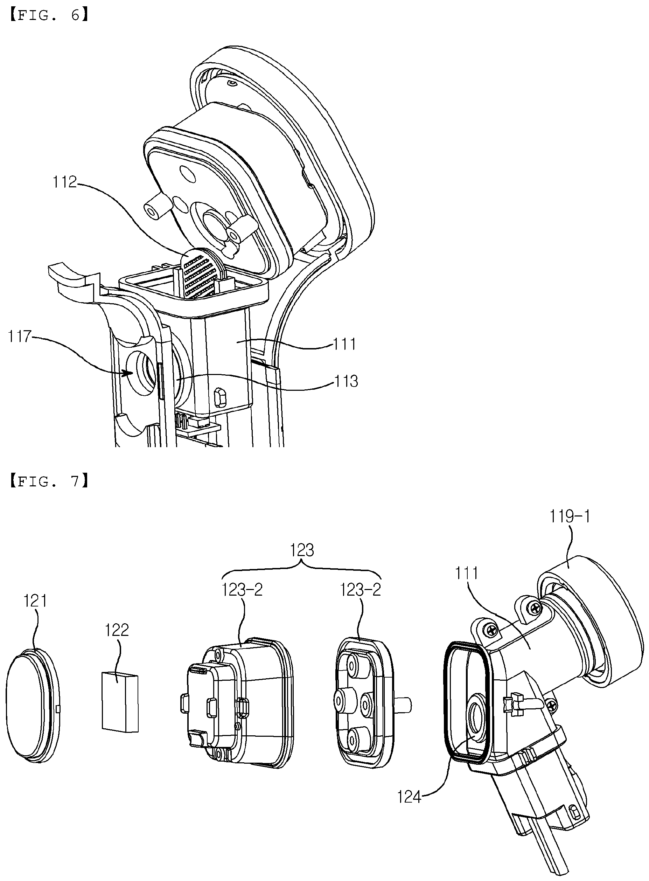

Referring to FIG. 6, the mist processing part 110 may be formed by including a water tank 111 containing raw water such as tap water, electrode modules 112 disposed inside the water tank 111 to transform the raw water to sterilization water through electrolysis, an ultrasonic oscillator 113 generating mists in fine particulate state by applying vibrations to the sterilization water generated by the electrode modules 112, and a mist outlet 117 through which mists generated by the ultrasonic oscillator 113 are released.

The structure, shape and material of water tank 111 may be variably formed but the present invention is not particularly limited thereto.

The head part 220 may be formed at one side thereof with a raw water inlet to allow the water to be poured into the water tank 111 of the mist processing part 110, and the water tank 111 may be formed in an approximate ' 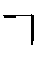 ' shape, or in an approximately right-angled bend, from the raw water inlet across an upper space of the handle part 210.

The raw water inlet formed at one side of the head part 220 may be opened/closed through a raw water cap 119-1 and a user may tight-seal the water tank 111 by closing the raw water cap 119-1 after opening the raw water cap 119-1 and pouring the raw water into the water tank 111.

Furthermore, the upper end of the handle part 210 may be formed with a mist outlet 117 toward a direction formed with the raw water inlet.

The electrode modules 112 may be disposed by being respectively spaced apart and formed by including one or more electrode plates respectively connected to a negative pole (-) and a positive pole (+), and can generate the sterilization water having sterilizing power by breaking down water molecules using the principle of underwater discharge.

That is, various negative ions (O⁻, O₃⁻, OH⁻, HOCl, H₂O₂) may be generated as a result of underwater plasma discharge through two electrode plates to allow the water to have the sterilizing power.

The said electrode modules 112 may be variably configured but the present invention is not particularly limited thereto.

The ultrasonic oscillator 113 may be so disposed as to encompass a surrounding of the mist outlet 117, whereby mists can be efficiently outputted.

That is, when an electric power is applied to the electrode modules 112 in response to control of the controller 140, the raw water can be transformed to sterilization water through electrolysis to allow generating sterilization water mists by way of the ultrasonic oscillator 113, and the generated sterilization water mists may be sprayed through the mist outlet 117.

FIG. 2 illustrates an example in which a switch for turning on or turning off the mist function is formed by way of a vertical sliding button 119-2 below the mist outlet 117.

Referring to FIG. 7, the temperature stimulation processing part 120 may be formed by including a skin contact member 121 for applying a temperature stimulation by being contacted to skin of a user and a thermoelement 122 for applying a heat to or cooling the skin contact member 121.

The structure, shape and material of skin contact member 121 may be variably formed. For example, the skin contact member 121 may be disposed with a plated shape formed with a material having adequate heat transfer properties.

The thermoelement 122 for temperature control may be variably formed and particularly formed using a peltier device 122. The role of the skin contact member 121 may be so disposed as to allow one surface of the peltier device 122 to directly perform.

The said one surface (external surface) of the peltier device 122 contacting the skin contact member 121 may be processed/treated with a particular temperature to stimulate the skin of a user.

In other words, when a current is applied to the peltier device 122 in response to the control of the controller 140, a relevant surface of the peltier device may be heated or cooled according to direction of the applied current.

Particularly, an inner surface of the peltier device 122 must be enabled to be adequately cooled for a stable operation including protection of the peltier device.

Various methods may be applied to implement the said cooling, and particularly, an inner surface of the peltier device 122 may be so disposed as to allow a thermal contact with the water stored in the water tank 111 of the mist processing part 110.

This means that the sterilization water stored in the water tank 111 of the mist processing part 110 may be used for cooling of the peltier device 122.

The thermal contact by the inner surface of the peltier device 122 with the water stored in the water tank 111 of the mist processing part 110 may be realized through a first heat dissipation part 124 of first material directly contacting the water stored in the water tank 111 of the mist processing part 110 and a second heat dissipation part 123 of second material interposed between the inner surface of the peltier device 122 and the first heat dissipation part 124.

That is, the inner surface of peltier device 122 and the water stored in the water tank 111 may be thermally contacted by at least two mutually different types of elements.

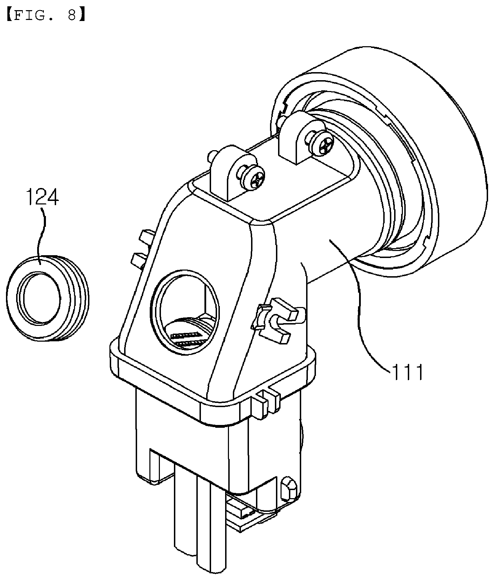

Referring to FIG. 8, the first heat dissipation part 124 may be installed through a hole formed on the water tank 111 to allow one surface to contact the sterilization water inside the water tank 111 and to simultaneously allow the other surface to contact the second heat dissipation part 123, wherein an area installed with the first heat dissipation part 124 must be well sealed using an O-ring lest the sterilization water be leaked.

The first heat dissipation part 124 may be disposed at an external one side (water tank side) of the second heat dissipation part 123 (see FIG. 6). The first heat dissipation part 124 may maximize the heat dissipation effect by being in contact with the sterilization water inside the water tank 124. Furthermore, the shape of the first heat dissipation part 124 is not particularly limited and the first heat dissipation part 124 may take a round shape, a polygonal shape or a star shape. The first heat dissipation part 124 may further enhance the heat dissipation effect when allowing an inner surface or an outlying area to have a plurality of protrusions.

Furthermore, a surface of the first heat dissipation part 124 contacting the sterilization water of the water tank 111 may be polluted by various types of ions. Therefore, the first material may be formed with a material less polluted than the second material by the sterilization water. As a detailed example, the first material of the first heat dissipation part 124 may be formed by including SUS material, and the second material of the second heat dissipation part 123 may be formed by including aluminum material.

In this case, the surface contacted by the sterilization water of the water tank 111 may have an effect of minimized oxidation.

As illustrated in FIG. 7, the thermal contact of the first heat dissipation part 124 and the second heat dissipation part 123 may be implemented at an area where the water tank 111 is curbed.

Meantime, the second heat dissipation part 123 may be organized by way of water cooling system that accommodates a cooling liquid therein in order to further enhance the heat dissipation function.

At this time, the second heat dissipation part 123 may be formed by including a main body 123-1 accommodating the cooling liquid and a lid 123-2, where a user may tight-seal by closing the lid 123-2 after pouring the cooling liquid into an inner space of main body 123-1.

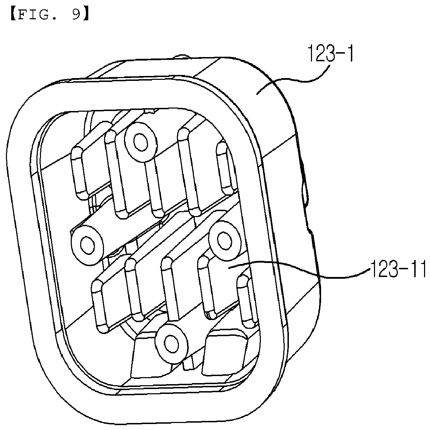

Furthermore, as shown in FIG. 9, a cooling efficiency may be further enhanced by forming a plurality of protrusions 123-11 inside an inner space of the main body 123-1.

Although the illustrated example has shown that the lid 123-2 of the second heat dissipation part 123 is fixed to the main body 123-1 using screws and bolts, an O-ring may be used for tight-sealing between the main body 123-1 and the lid 123-2.

The controller 140 can implement an overall control of the beauty device 100 in response to an operation command inputted through the input part 130. Methods for controlling the beauty device 100 using the controller 140 may be variably configured but the present invention is not particularly limited thereto.

For instance, the controller 140 may allow mists to be sprayed by driving the mist processing part 110 in response to ON/OFF signals of mist functions inputted through the input part 130, and may allow a current of an adequate direction to flow in the peltier device 122 in response to cool/warm stimulation set-up signals.

The housing may be disposed with a variety of display means to display the operation of beauty device 100. For example, an LED lamp may be disposed to display various information including, but not limited to, power ON/OFF states, an operation state of mist processing part 110, an operation state of temperature stimulation processing part 120 and a current cool/warm set-up state.

The power supply part 150 may be so configured as to transform a DC power to an AC power, and may be also configured to include a chargeable battery. The power supply part 150 may be variably formed by including a battery charging circuit as necessary.

Various constituent elements including a PCB forming an electric circuit and a battery may be installed within an inner space of the handle part 210.

Referring to FIGS. 2, 3 and 4, the beauty device 100 may include, for storage convenience, a cradle part 230 that performs a role of a rest.

The cradle part 230 may be so formed as to attach and detach a lower end of the handle part 210, and may include a function of charging a battery. At this time, a charging terminal may be disposed at a lower end of the handle part 210 and the cradle part 230 to allow a mutual electric contact when the handle part 210 is rested.

Referring to FIG. 3, the housing may be formed with a transparent window 171 along a surrounding of the skin contact member 121, and an inside of the transparent window 171 may be disposed with a light emitting part (not shown) to emit a predetermined light.

The said light emitting part may be formed using an LED, and the light outputted through the transparent window 171 may include various types of lights.

For example, the light outputted through the transparent window 171 may be used to display various operational states of the beauty device 100, may be used simply for ornamental purpose or may be used for skin beauty.

For example, a light with a wavelength of 590nm may be a wavelength that can reach a sebaceous gland located at a deepest side of skin, which is effective for removal of bodily wastes, skin whitening and skin regeneration.

Furthermore, a light with a wavelength of 850nm may demonstrate an improvement effect of damaged skin, a wrinkle improvement effect, a facial whitening effect, a redness removing effect and the like.

While the present invention has been described with reference to the particular illustrative embodiments, it is not to be restricted by the embodiments but only by the appended claims. It should be apparent to those skilled in the art that embodiments can be changed or modified without departing from the scope and spirit of the present invention.

### [Description of Reference Numerals]

- 100:: beauty device
- 110:: mist processing part
- 111:: water tank
- 112:: electrode module
- 113:: ultrasonic oscillator
- 117:: mist outlet
- 119-1:: raw water cap
- 120:: temperature stimulation processing part
- 121:: skin contact member
- 122:: thermoelement (peltier device)
- 123:: second heat dissipation part
- 123-1:: main body
- 123-2:: lid
- 124:: first heat dissipation part
- 130:: input part
- 140:: controller
- 150:: power supply part
- 171:: transparent window
- 210:: handle part
- 220:: head part
- 230:: cradle part

## Claims

1. A beauty device having a function of hydrogen sterilization water spray and a function of providing temperature stimulation to skins, the device comprising:
a mist processing (treatment) part generating sterilization water by electrolyzing water in a water tank and spraying the generated sterilization water in the form of mists;
a temperature stimulation processing (treatment) part applying temperature stimulation to skin using a thermoelement;
a power supply part supplying an electric power;
an input part receiving an operation command; and
a controller controlling the mist processing part and the temperature stimulation processing part in response to the operation command inputted through the input part, wherein a housing forming an external appearance may be formed in a portable manner.

2. The beauty device of claim 1, wherein the thermoelement includes a peltier device, wherein one surface of the peltier device is so configured as to thermally contact the water stored in the water tank of the mist processing part.

3. The beauty device of claim 2, wherein the thermal contact is realized through a first heat dissipation part of first material directly contacting the water stored in the water tank of the mist processing part and a second heat dissipation part of second material interposed between one surface of the peltier device and the first heat dissipation part, wherein the first material may be formed with a material less polluted than the second material by the sterilization water generated through the electrolysis.

4. The beauty device of claim 3, wherein the first material includes SUS material and the second material includes aluminum material.

5. The beauty device of claim 3, wherein the second heat dissipation part is so disposed as to accommodate a cooling liquid therein.

6. The beauty device of claim 5, wherein the second heat dissipation part is so disposed as to be tight-sealed by closing a lid after pouring the cooling liquid into an inner space.

7. The beauty device of any one of claims 1 to 6, wherein the housing includes a pillar-shaped handle part graspable by one hand of a user, and a head part protruded from an upper end of the handle part to both lateral surfaces, wherein one side of the head part is disposed with a raw water inlet to allow water to be poured into the water tank of the mist processing part, and the other side of the head part is disposed with a skin contact member configured to apply a temperature stimulation to skin by being contacted to the skin.

8. The beauty device of claim 7, wherein the water tank of the mist processing part is formed in the ' 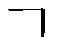 ' shape from the raw water inlet across an upper space of the handle part, and the upper end of the handle part is formed with a mist outlet toward a direction formed with the raw water inlet, and wherein the thermal contact of the first heat dissipation part and the second heat dissipation part is implemented at an area where the water tank is curbed.

9. The beauty device of claim 7, comprising a light emitting part formed with a transparent window along a surrounding of the skin contact member to output a predetermined light through the transparent window.

10. The beauty device of claim 9, wherein the light emitting part outputs a specific wavelength of light useable for skin beauty.
